# EUROPÄISCHE PATENTSCHRIFT

(11) **EP 2 682 139 B1**
(45) Veröffentlichungstag und Bekanntmachung des Hinweises auf die Patenterteilung: **24.12.2014**
(21) Anmeldenummer: 13174677.8
(22) Anmeldetag: 02.07.2013
(51) Int. Cl.: A61M 1/16, A61G 7/05, A61B 19/02, B01D 61/30

(54) **Haltevorrichtung und medizinisches Gerät zur extrakorporalen Blutbehandlung mit Haltevorrichtung**
Holding device and medical device for extracorporeal blood treatment with holding device
Dispositif de support et appareil médical pour le traitement extracorporel du sang doté du dispositif de support

(30) Priorität: 03.07.2012 DE 102012105912
(43) Veröffentlichungstag der Anmeldung: 08.01.2014
(73) Patentinhaber: B. Braun Avitum AG, 34212 Melsungen (DE)
(72) Erfinder: Schade, Andreas, 36199 Rotenburg (DE); Stenzel, Bruno, 26209 Hatten (DE)
(74) Vertreter: Winter, Brandl, Fürniss, Hübner, Röss, Kaiser, Polte - Partnerschaft mbB

(56) Entgegenhaltungen:
- EP-A1- 1 396 235
- WO-A1-96/40315
- DE-U1- 8 702 995
- FR-A1- 2 310 136
- US-A1- 2007 252 057

## Beschreibung

Die Erfindung betrifft eine Haltevorrichtung für ein medizinisches Gerät, umfassend einen Halter und ein zweites Bauteil, an welchem der Halter angebracht ist, wobei der Halter eine Aufnahme aufweist, an welcher ein Funktionsteil lösbar einbringbar ist.

Die Erfindung betrifft ferner ein medizinisches Gerät zur extrakorporalen Blutbehandlung mit einer solchen Haltevorrichtung.

Beispielsweise aus der WO 96/40315 A1 ist ein medizinisches Gerät zur extrakorporalen Blutbehandlung bekannt, das eine Haltevorrichtung zum Festklemmen eines Funktionsteils des medizinischen Geräts aufweist, wobei das Funktionsteil allerdings nicht lösbar in die Aufnahme des Halters einbringbar ist. Darüber hinaus ist in der EP 1 396 235 A1 eine chirurgische Konsole beschrieben, die allerdings keine Haltevorrichtung mit Halter und Aufnahme aufweist.

Bei medizinischen Geräten mit extrakorporalem Blutkreislauf, d.h. insbesondere bei Medizingeräten zur Blutreinigung, werden Filter eingesetzt, welche den Stoffausstauch ermöglichen. Diese Filter können am Medizintechnikgerät befestigt bzw. gehalten werden. Herkömmlicherweise sind diese Halter dabei aus Handlinggründen an exponierten Stellen angeordnet, sowie beweglich ausgeführt.

Häufig werden diese Halter als abnehmbares Zubehörteil ausgeführt und müssen vom Bediener des Gerätes an geeigneter Stelle montiert werden. Dies birgt jedoch die Gefahr, dass der Halter an ungeigneter Position angebracht bzw. in nicht vorgesehener Weise verwendet wird. Weiterhin besteht die Möglichkeit, dass der Halter vom Gerät entwendet wird und das Gerät dann nicht betrieben werden kann.

Ist der Halter dagegen starr mit dem Gehäuse des Geräts verbunden, besteht die Gefahr, dass der Halter beim Durchfahren von Engstellen wie beispielsweise Türrahmen abgefahren wird bzw. hierdurch Schäden am Medizintechnikgerät entstehen.

Aufgabe der Erfindung ist es daher, eine robuste Halterung für ein medizinisches Funktionsteil bereitzustellen, das Fehlbedienungen vorbeugt.

Aufgabe der Erfindung ist es ferner, ein medizinisches Gerät zur extrakorporalen Blutbehandlung mit einer solchen Halterung bereitzustellen.

Erfindungsgemäß wird diese Aufgabe durch eine Haltevorrichtung gemäß dem unabhängigen Anspruch 1 gelöst. Vorteilhafte Weiterbildungen der Haltevorrichtung ergeben sich aus den Unteransprüchen 2-8. Die Aufgabe wird ferner durch ein medizinisches Gerät zur extrakorporalen Blutbehandlung gemäß den Ansprüchen 9 und 10 gelöst.

Die erfindungsgemäße Haltevorrichtung für ein medizinisches Gerät umfasst dabei einen Halter und ein zweites Bauteil, an welchem der Halter angebracht ist bzw. mit welchem der Halter koppelbar ist, wobei der Halter eine Aufnahme aufweist, an welcher ein Funktionsteil lösbar einbringbar oder anschließbar ist. Das zweite Bauteil weist dabei eine Vertiefung auf, innerhalb der eine Halterung angebracht ist, die federbelastet gegenüber dem zweiten Bauteil bewegbar ist. Der Halter ist wiederum schwenkbar um eine Drehachse an der Halterung gelagert, wobei durch die federbelastete Halterung eine Zugkraft auf den Halter aufbringbar ist. Ferner ist der Halter bzw. die Vertiefung so ausgeformt, dass sich beim Schwenken des Halters um die Drehachse ein Schwenkabschnitt des Halters innerhalb der Vertiefung bewegt, während die Aufnahme aus der Vertiefung herausragt.

Durch diese Ausbildung einer Haltevorrichtung kann die Robustheit der Haltevorrichtung erhöht werden, denn die federnde Anbindung des Halters am zweiten Bauteil schützt beide Bauteile vor Beschädigungen. Ferner kann der Halter um eine Drehachse geschwenkt werden, was beabsichtigt, aber auch unbeabsichtigt bewirkt werden kann. Beabsichtigte Schwenkbewegungen ermöglichen die gezielte Positionierung des Halters in einer bestimmten Lage, und bei unbeabsichtigten Stößen verschwenkt der Halter, ohne dass es zu Rissen oder Brüchen in Bauteilen kommt.

Handelt es sich bei dem zweiten Bauteil beispielsweise um ein medizinisches Gerät zur extrakorporalen Blutbehandlung bzw. das Gehäuses eines solchen medizinischen Geräts, kann an der Haltevorrichtung ein Dialysator oder ein Halter zur Aufnahme eines Dialysators angebracht werden. Beim Durchfahren von Engstellen kann der Halter nicht abgefahren werden, und es kommt zu keinen Beschädigungen am Medizintechnikgerät. Dabei ragt nur die Aufnahme aus dem Gehäuse heraus, während sich der Schwenkabschnitt größtenteils innerhalb der Vertiefung im Gehäuse befindet und das Gehäuse so nach außen abschließen kann. Vorzugsweise wird der Bereich zwischen dem Schwenkabschnitt des Halters und der Vertiefung durch eine Abdeckung überbrückt oder sogar durch eine Dichtung abgedichtet.

Durch die erfindungsgemäße Haltevorrichtung kann ein Halter zur Aufnahme eines Funktionsteils an geeigneter Stelle in das Handlungsfeld eines Bedieners integriert werden, wodurch Fehlbedienungen vorgebeugt wird. Der Halter ist ferner so in das zweite Bauteil integriert, dass er nicht entwendet werden kann. Der Betrieb eines medizinischen Geräts kann somit nicht durch einen fehlenden Halter beeinträchtigt werden. Darüber hinaus besteht auch die Möglichkeit, weitere Elemente wie Schlauchhalter in den Halter zu integrieren, um beispielsweise Tropfkammern und/oder Schläuche aufnehmen zu können.

In einem Ausführungsbeispiel der Erfindung weist das zweite Bauteil Mittel auf, mit denen das Schwenken des Halters zwischen zwei definierten Endlagen begrenzbar ist. Hierzu können am zweiten Bauteil beispielsweise zwei Anschläge ausgeformt sein, die in den zwei definierten Endlagen mit jeweils einer Anschlagsfläche am Schwenkabschnitt des Halters in Kontakt kommen. Über die Endlagen hinaus kann der Halter somit nicht verschwenkt werden, wobei die Anschläge jedoch vorzugsweise so ausgebildet sind, dass sie die Schwenkbewegung des Halters stoppen, bevor er Kontakt zu Gehäuseteilen hat, die ansonsten beschädigt werden könnten. Hierbei handelt es sich insbesondere um den äußeren Rand der Vertiefung im Gehäuse, innerhalb der die Haltevorrichtung angebracht ist. Denn würde der Halter beim Schwenken an diesen Rand anschlagen, könnte es zu einem Bruch kommen.

Die schwenkbare Anbringung des Halters am zweiten Bauteil kann beispielsweise über einen Anbindungsbolzen bzw. Gelenkbolzen erfolgen, welcher durch ein Auge der Halterung geführt ist, wobei die beiden Endbereiche des Anbindungsbolzens jeweils in eine Aufnahme am zweiten Bauteil eingebracht sind. So kann eine Drehachse gebildet werden, um welche der Halter schwenkbar ist. In einem Ausführungsbeispiel der Erfindung befinden sich die Aufnahmen an einer Anschraubplatte, die lösbar am zweiten Bauteil angebracht ist. Eine solche Anschraubplatte hat insbesondere den Vorteil, dass die erforderliche Geometrie der Haltevorrichtung nicht im Gehäuse ausgebildet sein muss, was die Fertigung des Gehäuses erleichtert. Vielmehr kann die Anschraubplatte entsprechend ausgeformt und am Gehäuse angeschraubt sein. Dabei kann die Anschraubplatte auch aus einem anderen Material bestehen als das zweite Bauteil und/oder der Halter.

In einer weiteren Ausführungsform der Erfindung ist an der Haltevorrichtung eine Friktionsbremse bzw. Schleifbremse vorgesehen, mit welcher die Schwenkbewegung des Halters um die Drehachse bremsbar ist. Dies hat den Vorteil, dass sowohl beabsichtigte, als auch unbeabsichtigte Schwenkbewegungen gebremst werden, was das Risiko einer Bauteilbeschädigung weiter reduziert. Um eine Friktionsbremse zu realisieren, können am Schwenkabschnitt wenigstens zwei platten- oder scheibenförmige Bremselemente so ausgeformt sein, dass sie beim Schwenken des Halters permanent an der Halterung anliegen, wobei die Bremselemente auf zwei gegenüber liegenden Seiten an der Halterung anliegen und quer zur Drehachse verlaufen.

Von der Erfindung umfasst ist ferner ein medizinisches Gerät zur extrakorporalen Blutbehandlung, das ein Gehäuse und eine Haltevorrichtung mit einem Halter zur Aufnahme eines Funktionsteils des medizinischen Geräts aufweist, wobei die Haltevorrichtung erfindungsgemäß ausgebildet ist. Das aufzunehmende Funktionsteil kann dann beispielsweise ein Dialysator oder ein weiterer Halter zur Aufnahme des Dialysators sein. Ein solches medizinisches Gerät kann die Vorteile der erfindungsgemäßen Haltevorrichtung nutzen und ist insbesondere gegen Beschädigungen geschützt. Die Haltevorrichtung kann jedoch auch an anderen medizinischen Geräten vorgesehen werden, bei denen eine robuste Halterung für ein Funktionsteil bereitgestellt werden soll.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1: einen schematischen Schnitt quer durch die Schwenkachse eines Ausführungsbeispiels der erfindungsgemäßen Haltervorrichtung, wobei der Halter in einer ersten Endlage anschlägt;
- Fig. 2: einen schematischen Schnitt gemäß Fig. 1, wobei der Halter in einer zweiten Endlage anschlägt; und
- Fig. 3: einen schematischen Schnitt längs der Schwenkachse des Halters gemäß Fig. 1.

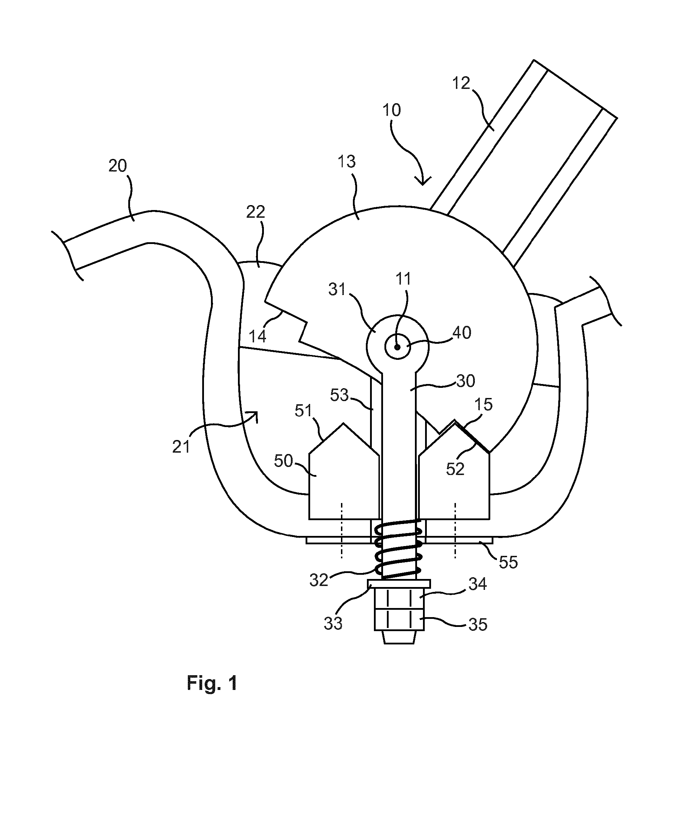

Fig. 1 zeigt ein Ausführungsbeispiel einer erfindungsgemäßen Haltevorrichtung, die in ein zweites Bauteil 20 integriert ist. Bei dem zweiten Bauteil 20 handelt es sich beispielsweise um das Gehäuse eines medizinischen Geräts, wobei es sich insbesondere um ein medizinisches Gerät zur extrakorporalen Blutbehandlung handeln kann, an dem für eine Therapie ein Funktionsteil wie ein Filter bzw. Dialysator angebracht werden. Die Erfindung soll durch die Figuren anhand eines solchen Geräts zur extrakorporalen Blutbehandlung beschrieben werden, aber die Haltevorrichtung kann auch für andere medizinische Geräte eingesetzt werden, an denen Funktionsteile lösbar angebracht werden sollen.

Fig. 1 zeigt dabei einen schematischen Schnitt quer durch die Schwenkachse 11 eines Halters 10, wobei nur der Ausschnitt des Gehäuses 20 dargestellt ist, in dem die Halterung 30 angebracht ist. Dabei kann es sich beispielsweise um den Eckbereich eines Gehäuses handeln.

Die Haltevorrichtung umfasst den Halter 10, der dazu ausgebildet ist, direkt oder indirekt ein Funktionsteil des medizinischen Geräts aufzunehmen. Dazu ist am Halter 10 eine Aufnahme bzw. ein Aufnahmeabschnitt 12 vorgesehen, die im dargestellten Ausführungsbeispiel so ausgebildet ist, dass ein Filterhalter in die hohlzylindrische Aufnahme 12 einschiebbar ist. In diesen Filterhalter kann dann der Dialysator eingebracht werden. Vorzugsweise ist ein solcher Filterhalter axial innerhalb der Aufnahme 12 arretierbar, jedoch gleichzeitig drehbar gelagert, so dass der Filterhalter innerhalb der Aufnahme 12 gedreht werden kann.

Die Aufnahme 12 kann jedoch auch direkt als Filterhalter ausgeführt sein, in den ein Dialysator lösbar einbringbar ist, so dass der Filter nach einer Benutzung wieder aus der Haltevorrichtung entnommen werden kann. Der Halter 10 dabei besteht vorzugsweise aus Kunststoff. Ferner können am Halter 10 ergänzend ein Kammerhalter und/oder ein Schlauchhalter angebracht oder integral ausgeformt sein.

Die Haltevorrichtung umfasst eine Vertiefung 21, in welche der Halter 10 schwenkbar integriert ist. Die Vertiefung 21 befindet sich in dem Ausführungsbeispiel der Figuren in einem Eckbereich des Gehäuses 20, so dass diese Ecke als Haltevorrichtung ausgeformt ist. Dabei ist der Halter 10 aufgeteilt in einen Schwenkabschnitt 13 und die Aufnahme 12, wobei sich der Schwenkabschnitt 13 innerhalb oder zumindest größtenteils innerhalb der Vertiefung 21 befindet, während die Aufnahme 12 aus der Vertiefung 21 herausragt. Der Schwenkabschnitt 13 schließt über eine Abdeckung 22 gegenüber dem Gehäuse 20 ab. Diese Abdeckung kann auch abdichtend wirken, so dass sie auch als Abdichtung angesehen werden kann.

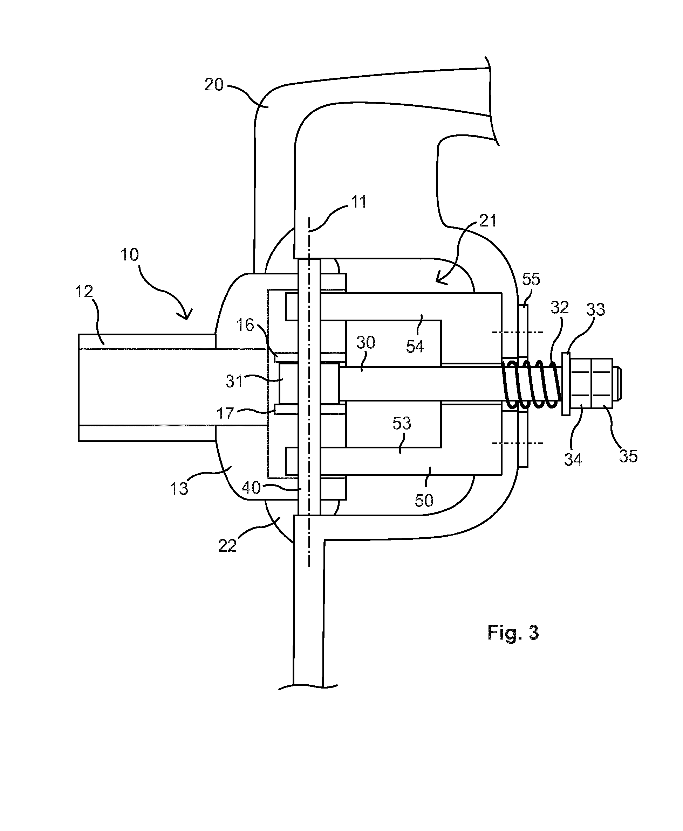

Der Halter 10 ist schwenkbar über eine Drehachse 11 an einer Halterung 30 gelagert, die wiederum federbelastet gegenüber dem Gehäuse 20 bewegbar ist. Dabei ist die Halterung beispielsweise als Augenschraube 30 ausgeführt, durch deren Auge 31 ein Anbindungsbolzen bzw. Gelenkbolzen 40 geführt ist. Die Anbindung dieses Anbindungsbolzens 40 am Gehäuse 20 ist in einer anderen Ansicht auch aus Fig. 3 ersichtlich.

Die Augenschraube 30 ist in einem Ausführungsbeispiel der Erfindung mit ihrem freien Ende durch eine Bohrung in einer Anschraubplatte bzw. Montageteil 50 geführt, welche am Gehäuse 20 angebracht ist, und ragt in eine Öffnung in der Vertiefung 21. Dabei kann von innen ein plattenförmiges Blechteil 55 an dem Gehäuse 20 positioniert werden, so dass eine Schraubverbindung durch dieses Blechteil 55 und das Gehäuse 20 in die Anschraubplatte 50 geführt werden kann. Das Gehäuse 20 wird so zwischen der Anschraubplatte 50 und dem Blechteil 55 eingeklemmt. Durch das Blechteil 55 wird unter Umständen je nach Material der Anschraubplatte 50 und des Gehäuses 20 die Stabilität der Verbindung verbessert. Die Verbindung zwischen der Anschraubplatte 50 und dem Gehäuse 20 kann jedoch auch auf andere Arten ausgeführt sein, so dass das Gehäuse 20 vorzugsweise in diesem Bereich entsprechend ausgeformt ist, um die Anschraubplatte 50 geeignet anbringen zu können.

Auf der anderen Seite der Anschraubplatte 50 ist die Augenschraube 30 von einer Druckfeder 32 umgeben, welche zusammen mit einem Anschlag am Ende der Augenschraube 30 eine Zugkraft auf die Augenschraube 30 aufbringt, welche den Halter 10 an das Gehäuse 20 heranzieht, da die Feder 32 den Anschlag von der Anschraubplatte 50 wegdrückt. Der Anschlag kann dabei beispielsweise durch eine Scheibe 33 und zwei Kontermuttern 34 und 35 gebildet werden, die auf das Gewinde der Augenschraube 30 aufgeschraubt sind. Auch eine selbstsichernde Mutter könnte hierzu verwendet werden. Der Anschlag kann auch fest an der Halterung 30 ausgeformt sein, jedoch bieten Muttern den Vorteil einer besseren Justierbarkeit der Federspannung und erleichtern die Montage der Haltevorrichtung.

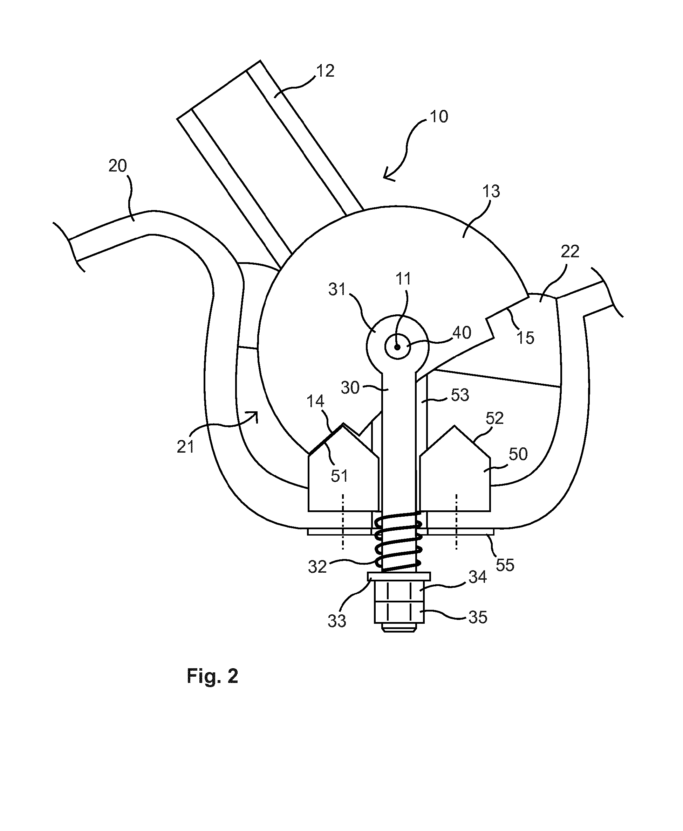

Dabei kann die Feder 32 auf der anderen Seite an dem Blechteil 55, dem Gehäuse 20 oder der Anschraubplatte 50 anliegen. Dies hängt im Wesentlichen von der Art der Verbindung zwischen Anschraubplatte 50 und Gehäuse 20 ab, wobei es sich als vorteilhaft erwiesen hat, wenn die Feder 32 wie in Fig. 2 durch das Blechteil 55 und das Gehäuse 20 hindurchgeführt ist und an der Anschraubplatte 50 anliegt. Die Baugruppe kann dann vormontiert und mit der Augenschraube 30 durch die Aussparung innerhalb der Anschraubplatte 50 geführt werden. Anschließend wird im Innern des Gehäuses das Blechteil 55 angelegt und mit Schrauben am Gehäuse 20 befestigt, woraufhin die Feder 32 auf der Augenschraube 30 anbringbar ist.

Die Anschraubplatte 50 ist ferner so ausgeformt, dass sie zwei Anschläge 51 und 52 in Form von schrägen Flächen aufweist. Der Schwenkabschnitt 13 des Halters 10 hingegen weist entsprechende Anschlagsflächen 14 und 15 auf, welche beim Schwenken des Halters 10 in zwei definierten Endlagen in Kontakt mit den Anschlägen 51 und 52 kommen. Dazu können die Anschlagsflächen 14, 15 an den äußeren Kanten des Schwenkbereichs 13 in diesen eingelassen sein, so dass sich dort jeweils eine Einbuchtung ergibt, in die sich eine entsprechende Kante der Anschlagplatte 50 beim Anschlagen einfügt.

Fig. 1 zeigt den Halter 10 in einer ersten definierten Endlage, in welcher er mit der Anschlagfläche 15 an dem Anschlag 52 der Anschraubplatte Montageteil 50 anliegt. Bringt man Druck gegen den Halter 10 auf bzw. ein Drehmoment an dem Halter 10 an, kann dieser um die Drehachse 11 bis zur anderen definierten Endlage schwenken, die in Fig. 2 dargestellt ist. Dabei schützt die federnde Anbindung des Halters 10 das Gehäuse 20 und den Halter 10 vor Beschädigungen. Ferner kann der Schwenkabschnitt 13 halbschalenförmig ausgebildet sein, so dass er in etwa der Außenkontur des Gehäuses 20 folgt und das Gehäuse 20 so nach außen abschließt.

Fig. 3 zeigt einen schematischen Schnitt längs der Drehachse 11, um insbesondere die Halterung des Halters 10 an der Anschraubplatte 50 und damit an dem Gehäuse 20 zu verdeutlichen. Die Augenschraube 30 verläuft durch die Anschraubplatte 50 und weist auf der anderen Seite das Auge 31 auf, durch welches der Anbindungsbolzen 40 geführt ist. Die Enden des Anbindungsbolzens 40 sind dabei in zwei Aufnahmen 53 und 54, die an der Anschraubplatte 50 ausgeformt sind und sich auf gegenüber liegende Seiten der Augenschraube 30 befinden, geführt. Eine untere dieser Aufnahmen 53 ist auch im Querschnitt der Figuren 1 und 2 gezeigt.

Ferner ist eine Friktionsbremse bzw. Schleifbremse vorgesehen, durch welche das Schwenken des Halters 10 um die Drehachse 11 bremsbar ist. Diese Friktionsbremse kann durch jegliche Bauteile am Halter 10 gebildet werden, die an fest stehenden Bauteilen anliegen und so durch Reibung eine entsprechende Bremswirkung entfalten. Im Ausführungsbeispiel der Figuren wird dies durch zwei plattenförmige Bremselemente 16 und 17 erreicht, die am Schwenkabschnitt 13 des Halters 10 ausgeformt bzw. angebracht sind. Die Bremselemente 16, 17 liegen an gegenüber liegenden Seiten an dem Auge 31 der Augenschraube 30 an, wobei sie quer zur Drehachse 11 verlaufen. Die zum Auge 31 gewandten Flächen der Bremselemente 16, 17 können mit einer Oberfläche versehen sein, welche den Reibungskoeffizienten erhöht.

### Bezugszeichenliste:

- 10: Halter
- 11: Drehachse
- 12: Aufnahme, Filterhalter, Dialysatorhalter
- 13: Schwenkabschnitt
- 14,15: Anschlagfläche
- 16,17: Bremselement, Platte
- 20: Zweites Bauteil, Gehäuse, medizinisches Gerät
- 21: Vertiefung
- 22: Abdeckung, Dichtung
- 30: Halterung, Augenschraube
- 31: Auge
- 32: Feder, Druckfeder
- 33: Scheibe
- 34,35: Mutter
- 40: Anbindungsbolzen, Gelenknbolzen
- 50: Anschraubplatte; Montageteil
- 51,52: Anschlag
- 53,54: Aufnahme
- 55: Blechteil

## Patentansprüche

1. Haltevorrichtung für ein medizinisches Gerät, umfassend einen Halter (10) und ein zweites Bauteil (20), mit welchem der Halter (10) gekoppelt oder koppelbar ist, wobei der Halter (10) eine Aufnahme (12) aufweist, an welcher ein Funktionsteil lösbar einbringbar ist,
**dadurch gekennzeichnet, dass**
der Halter (10) ferner einen Schwenkabschnitt (13) aufweist, über welchen der Halter (10) und das in die Aufnahme (12) einbringbare Funktionsteil schwenkbar um eine Drehachse (11) an einer Halterung (30) des zweiten Bauteils (20) gelagert sind, wobei die Halterung (30) federbelastet gegenüber dem zweiten Bauteil (20) bewegbar ist; und
das zweite Bauteil (20) eine Vertiefung (21) aufweist, innerhalb der der Schwenkabschnitt (13) und die Halterung (30) derart angeordnet sind, dass beim Schwenken des Halters (10) um die Drehachse (11) lediglich die Aufnahme (12) aus der Vertiefung (21) herausragt.

2. Haltevorrichtung nach Anspruch 1,
**dadurch gekennzeichnet, dass** ein Bereich zwischen dem Schwenkabschnitt (13) des Halters (10) und der Vertiefung (21) durch eine Abdeckung (22) überbrückt ist.

3. Haltevorrichtung nach einem oder beiden der Ansprüche 1 und 2,
**dadurch gekennzeichnet, dass** das zweite Bauteil (20) Mittel (51, 52) aufweist, mit denen das Schwenken des Halters (10) zwischen zwei definierten Endlagen begrenzbar ist, insbesondere der Halter (10) in den Endlagen jeweils arretiert werden kann.

4. Haltevorrichtung nach Anspruch 3,
**dadurch gekennzeichnet, dass** am zweiten Bauteil (20) zwei Anschläge (51, 52) ausgeformt sind, die in den zwei definierten Endlagen mit jeweils einer Anschlagsfläche (14, 15) am Schwenkabschnitt (13) des Halters (10) in Kontakt kommen.

5. Haltevorrichtung nach einem oder mehreren der Ansprüche 1 bis 4,
**dadurch gekennzeichnet, dass** der Halter (10) über einen Gelenkbolzen (40) schwenkbar am zweiten Bauteil (20) angebracht ist, welcher durch ein Auge (31) der Halterung (30) geführt ist, wobei die beiden Endbereiche des Anbindungsbolzens (40) jeweils in eine Aufnahme (53, 54) am zweiten Bauteil (20) eingebracht sind.

6. Haltevorrichtung nach Anspruch 5,
**dadurch gekennzeichnet, dass** sich die Aufnahmen (53, 54) an einem Montageteil (50) befinden, das lösbar am zweiten Bauteil (20) angebracht ist.

7. Haltevorrichtung nach einem oder mehreren der Ansprüche 1 bis 6,
**dadurch gekennzeichnet, dass** eine Schleifbremse vorgesehen ist, mit welcher die Schwenkbewegung des Halters (10) um die Drehachse (11) bremsbar ist.

8. Haltevorrichtung nach Anspruch 7,
**dadurch gekennzeichnet, dass** am Schwenkabschnitt (13) wenigstens zwei Bremselemente (16; 17) so ausgeformt sind, dass sie beim Schwenken des Halters (10) permanent an der Halterung (30) anliegen, wobei die Bremselemente (16, 17) auf zwei gegenüber liegenden Seiten an der Halterung (30) anliegen.

9. Medizinisches Gerät zur extrakorporalen Blutbehandlung, umfassend ein Gehäuse (20) und eine Haltevorrichtung gemäß einem oder mehreren der Ansprüche 1 bis 8.

10. Medizinisches Gerät nach Anspruch 9,
**dadurch gekennzeichnet, dass** das Funktionsteil ein Dialysator oder ein Halter zur Aufnahme eines Dialysators ist.

## Claims

1. A holding device for a medical device comprising a holder (10) and a second component part (20) by which the holder (10) is coupled or adapted to be coupled, the holder (10) including a receiving portion (12) to which a functional part can be detachably introduced, wherein
the holder (10) further includes a pivot portion (13) by which the holder (10) and the functional part which can be introduced into the receiving portion (12) are pivotably mounted to a support (30) of the second component part (20) so as to be pivotable about an axis of rotation (11), the support (30) being movable relative to the second component part (20) in a spring-loaded manner; and
the second component part (20) includes a recess (21) inside which the pivot portion (13) and the support (30) are arranged so that when the holder (10) is pivoted about the axis of rotation (11) merely the receiving portion (12) protrudes from the recess (21).

2. The holding device according to claim 1,
wherein an area between the pivot portion (13) of the holder (10) and the recess (21) is bridged by a cover (22).

3. The holding device according to one or both of claims 1 or 2,
wherein the second component part (20) comprises means (51, 52) by which pivoting of the holder (10) can be delimited between two defined end positions, especially the holder (10) can be locked in each of the end positions.

4. The holding device according to claim 3,
wherein two stops (51, 52) which at the two defined end positions contact respective stop faces (14, 15) at the pivot portion (13) of the holder (10) are formed at the second component part (20).

5. The holding device according to one or more of claims 1 to 4,
wherein the holder (10) is pivotably mounted to the second component part (20) via a pivot bolt (40) guided through an eye (31) of the support (30), wherein each of the two end portions of the connecting bolt (40) is introduced in a respective receiving portion (53, 54) at the second component part (20).

6. The holding device according to claim 5,
wherein the receiving portions (53, 54) are provided at a mounting member (50) detachably arranged on the second component part (20).

7. The holding device according to one or more of claims 1 to 6,
wherein a friction brake is provided for decelerating the pivoting motion of the holder (10) about the axis of rotation (11).

8. The holding device according to claim 7,
wherein at the pivot portion (13) at least two braking elements (16; 17) are formed such that they are in permanent contact with the support (30) upon pivoting the holder (10), wherein the braking elements (16, 17) are adjacent to the support (30) on two opposed sides.

9. A medical device for extracorporeal blood treatment comprising a housing (20) and a holding device according to one or more of claims 1 to 8.

10. The medical device according to claim 9,
wherein the functional member is a dialyser or a holder for accommodating a dialyser.

## Revendications

1. Dispositif de support pour un appareil médical, comprenant un support (10) et un deuxième élément (20) avec lequel le support (10) est couplé ou peut être couplé, dans lequel le support (10) présente un réceptacle (12) sur lequel un élément fonctionnel peut être inséré de façon amovible,
**caractérisé en ce que**
le support (10) présente en outre une portion de pivotement (13) par le biais de laquelle le support (10) et l'élément fonctionnel pouvant être insérée sur le réceptacle (12) sont montés de façon à pouvoir pivoter autour d'un axe de rotation (11) prévu sur un élément de fixation (30) du deuxième élément (20), l'élément de fixation (30) pouvant être déplacé sous l'action d'un ressort par rapport au deuxième élément (20) ; et
le deuxième élément (20) présente une cavité (21) à l'intérieur de laquelle la portion de pivotement (13) et l'élément de fixation (30) sont agencés de telle sorte que, lors du pivotement du support (10) autour de l'axe de rotation (11), seul le réceptacle (12) dépasse de la cavité (21).

2. Dispositif de support selon la revendication 1,
**caractérisé en ce qu'**une zone entre la portion de pivotement (13) du support (10) et la cavité (21) est couverte par un cache (22).

3. Dispositif de support selon l'une ou les deux des revendications 1 et 2,
**caractérisé en ce que** le deuxième élément (20) présente des moyens (51, 52) par l'intermédiaire desquels le pivotement du support (10) peut être limité entre deux positions extrêmes définies, en particulier par l'intermédiaire desquels le support (10) peut être arrêté dans chacune des positions extrêmes.

4. Dispositif de support selon la revendication 3,
**caractérisé en ce que** deux butées (51, 52) sont prévues sur le deuxième élément (20), lesquelles entrent en contact dans chacune des deux positions extrêmes définies avec une surface de butée respective (14, 15) prévue sur le segment de pivotement (13) du support (10).

5. Dispositif de support selon une ou plusieurs des revendications 1 à 4,
**caractérisé en ce que** le support (10) est monté de façon à pouvoir pivoter sur le deuxième élément (20) par un axe d'articulation (40) qui passe par un billet (31) de l'élément de fixation (30), les deux extrémités terminales de l'axe de liaison (40) étant chacune insérées dans un logement respectif (53, 54) prévu sur le deuxième élément (20).

6. Dispositif de support selon la revendication 5,
**caractérisé en ce que** les logements (53, 54) sont prévus sur une portion de montage (50) qui est rapportée de manière amovible sur le deuxième élément (20).

7. Dispositif de support selon une ou plusieurs des revendications 1 à 6,
**caractérisé en ce qu'**un frein à disque est prévu, par l'intermédiaire duquel le mouvement de pivotement du support (10) autour de l'axe de rotation (11) peut être freiné.

8. Dispositif de support selon la revendication 7,
**caractérisé en ce qu'**au moins deux éléments de freinage (16 ; 17) sont prévus sur la portion de pivotement (13) de telle sorte qu'ils s'appuient en permanence sur l'élément de fixation (30) lors du pivotement du support (10), les éléments de freinage (16, 17) s'appuyant sur deux côtés opposés sur l'élément de fixation (30).

9. Appareil médical de traitement extracorporel du sang, comprenant un boîtier (20) et un dispositif de support selon une ou plusieurs des revendications 1 à 8.

10. Appareil médical selon la revendication 9,
**caractérisé en ce que** l'élément fonctionnel est un dialyseur ou un support pour recevoir un dialyseur.
